# EUROPEAN PATENT APPLICATION

(11) **EP 4 059 575 A1**
(43) Date of publication of application: **21.09.2022**
(21) Application number: 21162942.3
(22) Date of filing: 16.03.2021
(51) Int. Cl.: A61Q 19/10, A61Q 5/02, A61Q 5/12, A61K 8/02, A61K 8/60, A61K 8/92, A61K 8/44, A61K 8/73, A61K 8/20, A61K 8/362, A61K 8/24, A61K 8/9794, A61K 8/46, C11D 1/12, C11D 1/14, C11D 1/37, C11D 3/04

(54) **CLEANSING COMPOSITION**

(71) Applicant: Li-iL GmbH Arzneimittel, Arneibäder, 01139 Dresden (DE)
(72) Inventor: GRASSE, Christiane, 99425 Weimar (DE); HARTENSTEIN, Katharina, 01159 Dresden (DE)
(74) Representative: Wallinger Ricker Schlotter Tostmann

(57) **Abstract**

The present invention relates to a powdery composition, comprising at least one substrate, wherein the substrate is not a salt, at least one surfactant, at least one viscosity adjusting agent as well as to an aqueous composition derived there from.

## Description

### FIELD OF THE INVENTION

The present invention relates to a powdery composition, an aqueous cleansing composition, as well as the manufacturing methods and use thereof.

### BACKGROUND OF THE INVENTION

Commercially available cleansing compositions that are useful for cleansing the skin and hair regularly contain very high amounts of water (about 60 to 80% by weight) in order providing the gel-like consistency which makes these cleansing compositions ready for use for the costumer.

However, the water contained in the cleansing composition per se does not have any cleansing or skin/hair caring properties. This means, however, that when it comes to shipping the ready-for-use cleansing compositions from e.g. the manufacturer site to distributors or supermarkets a lot of "useless" water is transported, and thus, ultimately a lot of energy (e.g. in form of fuel) is consumed for just shipping water.

In times where the climate changes drastically, however, there are several attempts to avoid shipping of "useless" water, e.g. cleansing compositions in form of a bar of soap.

However, such products are often not accepted by the end costumer, since the end costumers report that the used gel-like consistency is missed. Another point which is frequently addressed is that the cleansing as well as skin/hair caring properties of the cleansing compositions which are designed as soap differ from the gel-like ones.

### OBJECTS OF THE INVENTION

Thus, it is an object of the present invention to provide a composition which on the one hand contains no "useless" water, but on the other hand provides the same consistency, cleansing as well as skin/hair caring properties as the commercially available gel-like, ready-to-use cleansing compositions.

### SUMMARY OF THE INVENTION

The present invention encompasses the following items:
Item 1: Powdery composition, comprising at least one substrate, wherein the substrate is not a salt; at least one surfactant, wherein the amount of the at least one surfactant is at most 70 g, based on 100 g of the total powdery composition; at least one viscosity adjusting agent; wherein all components of the composition are present in the composition in powder form and add up to 100 wt.-% of the powdery composition.
Item 2: Composition according to item 1, wherein all components are water soluble.
Item 3: Composition according to at least one of the items 1 or 2, wherein the composition comprises one or more further components which are selected from the list comprising fragrances, essential oils, dyes, pH adjusting agents, chelating agents, conditioning agents, active ingredients, emollients, moisturizing agents, pearlescent agents, opacifying agents, emulsifier, absorbents, preserving agents, antioxidants.
Item 4: Composition according to at least one of the items 1 to 3, wherein the at least one substrate is selected from lactose, silica, mannite, xylite, diatomite, cellulose and derivates thereof, monocrystalline cellulose, modified cellulose, trimethyl glycine, saccharose, monosaccharide, polysaccharide, cyclodextrin, native starch, chemically modified starch, thermal modified starch, sorbitol, polyvinylpyrrolidone, silicate, or mixtures thereof.
Item 5: Composition according to at least one of the items 1 to 4, wherein the at least one viscosity adjusting agent present in the powdery composition according to items 1 to 4 is selected from natural polymers, synthetic thickeners, ethers, alkoxylated alcohols, hydrophilic colloids or derivatives thereof, salts of monovalent or divalent cations or mixtures thereof.
Item 6: Composition according to at least one of the items 1 to 5, wherein the at least one surfactant is selected from isethionate, amino acid, sulfonic acid, sulfuric acid ester, alkyl sulfate, alkylether sulfate, taurate, sulfosuccinate, sulfosuccinamate, carboxylate, glucosides, amphoacetates, amide, alkanol amide, or mixtures thereof.
Item 7: Method for manufacturing the composition according to at least one of items 1 to 6, wherein the method comprises a mixing step, wherein the components of the composition are mixed together.
Item 8: Method according to item 7 wherein prior to the mixing step an uptake step is conducted, wherein one or more of the components being present in liquid form are up-taken onto the at least one substrate and transferred into a powder form.
Item 9: Method for manufacturing an aqueous cleansing composition by mixing the powdery composition as claimed in at least one of the items 1 to 6 or by mixing the powdery composition as obtained by the method as claimed in at least one of the items 7 or 8 with water, wherein the water is selected from deionized water, purified water, mineral water or tap water, or a mixture thereof.
Item 10: Method as claimed in item 9 wherein the mixing of water and the powdery composition is done by shaking and/or stirring.
Item 11: Method as claimed in at least one of items 9 or 10, wherein the weight ratio of powdery composition : added water is 1:2.5 to 1:4.5.
Item 12: Aqueous cleansing composition comprising the powdery composition according to at least one of the items 1 to 6 in dissolved form or comprising the powdery composition as obtained by the method as claimed in at least one of the items 7 or 8 in dissolved form; or aqueous cleansing composition as obtained by the method as claimed in at least one of the items 9 to 11.
Item 13: Aqueous cleansing composition according to item 12, wherein the amount of the at least one surfactant is not more than 14 g wherein the amount is based on 100 g of the total aqueous cleansing composition, if the weight ratio of powdery composition : added water is 1:4.
Item 14: Aqueous cleansing composition according to at least one of the items 12 or 13, wherein the aqueous cleansing composition is selected from the group consisting of shampoo, 2-in-1 shampoo conditioner, shower gel, shower cream, hair and body washes, liquid hand soaps, facial cleanser or combinations thereof for human beings and for animals.
Item 15: Aqueous cleansing composition according to at least one of the items 12 to 14, wherein the pH of the aqueous cleansing composition is between 4.0 to 6.0 or between 5.0 to 7.5

### DETAILED DESCRIPTION OF THE INVENTION

The above addressed object is solved by the powdery composition according to the invention, wherein the powdery composition comprises at least one substrate, wherein the substrate is not a salt, at least one surfactant, wherein the amount of the at least one surfactant is at most 70 g based on 100 g of the total composition, at least one viscosity adjusting agent, wherein all components of the composition are present in the composition in powder form and add up to 100 wt.-% of the powdery composition.

The powdery composition according to the invention has the advantage that all components are in powder form and thus, no "useless" water needs to be transported. Another advantage of the powdery composition according to the invention is that the costumer just needs to add water at home to the powdery composition and thus obtains a gel-like, ready-to-use aqueous cleansing composition which is comparable or even better in terms of cleansing efficiency, viscosity, and hair/skin caring properties, pH-adjustment, fragrance variety and foaming properties to the commercially available aqueous gel-like, ready-to-use cleansing compositions.

The term "powdery composition" as used within the context of the present application refers to a composition comprising or consisting of one or more substrates, one or more surfactants, one or more viscosity adjusting agents. Further optional components which may be present in the powdery composition according to the invention are selected from one or more fragrances, one or more essential oils, one or more chelating agents, one or more active ingredients, one or more emulsifier, one or more emollients, one or more absorbents, one or more dyes, one or more pH adjusting agents, one or more conditioning agents, one or more moisturizing agents, one or more pearlescent agents, one or more opacifying agents, one or more preserving agents, one or more antioxidants and mixtures thereof. Among the mentioned components, some components may have a two- or more fold action: E.g. starch may act on the one hand as substrate and at the same time also as absorbent.

Said components are present in the powdery composition according to the invention in powder form. This in turn means that the powdery composition according to the invention does not contain any component in liquid form. The powdery composition according to the invention has a residual moisture content of not more than 2 % by weight, preferably not more than 1.8 % by weight, even more preferably not more than 1.5 % by weight based on the total weight of the powdery composition, wherein the residual moisture content is measured according to DIN 51718.

The terms "powdery" and "in powder form" as used within the context of the present application means that the components are present in the powdery composition according to the invention as solid particles. The D50 particle size (median) of the solid particles can vary between 50 µm up to 700 µm, wherein the particle size is measured using a Mastersizer 2000 at 3 bar. The powdery composition according to the invention can be designed as powder having free-flowing properties. In such a case, where the powdery composition according to the invention is designed as powder having free-flowing properties, the solid particles are essentially not adhered together and/or do not stick together and thus, the free-flowing properties are provided. The powdery composition according to the invention can also be designed as powder having no free-flowing properties. In such a case, where the powdery composition according to the invention has no free-flowing properties, the solid particles are adhered and/or stuck together by physical (e.g. melting together the solid particles), chemical (e.g. by gluing together the sold particles) and/or mechanical means (e.g. by pressing together the solid particles) or a combination thereof. The powdery composition may then e.g. provided as pallet. The powdery composition according to the invention, independently of the fact if it is provided as powder having free-flowing properties or no free-flowing properties, may be enwrapped by a shell which easily dissolves when being poured into water and then releasing the powdery composition according to the invention. It is also possible that the powdery composition according to the invention, independently of the fact if it is provided as powder having free-flowing properties or no free-flowing properties, may be provided as such, i.e. without any enwrapping shell.

In addition, the components of the powdery composition according to the invention are all at least partly, preferably completely soluble in water. When water is added to the powdery composition according to the invention, the components of the powdery composition are dissolved in the added water and thus, a gel-like, ready-to-use aqueous cleansing composition is obtained, having at least comparable, preferably improved cleansing efficiency, viscosity, and hair/skin caring properties like commercially available gel-like, ready-to-use aqueous cleansing compositions.

Further, it is preferred that the components of the powdery composition according to the invention are based on natural components or are based on components of natural origin, are vegan and are free of any harmful or problematic substances. In particular, the powdery composition according to the invention contains no ethoxylated components and/or no mineral oil based components and/or no EDTA and / or no silicon oils and/or silicon-oil based compounds and/or no components of animal origin and/or no genetically modified components and/or UV-filter (e.g. benzophenones) and/or no butylhydoxyanisol (BHA) and/or no butylhydroxytoluol (BHT) and/or no toluene and/or no benzene and/or no tetrabutyl ethylidinebisphenole and/or no halogenorgano components and/or no parabens and/or no methylisothiazolinone and/or no aromatic amines and/or no azo dyes and/or no PEG / PEG derivatives and/or no cashmerane and/or no majantol, and/or no polycyclic or nitro-containing musk derivatives and/or no lilial and/or no gernyl nitrile and/or no phthalates and/or no vetiveryl acetate and/or no methyl chloro isothiazolinone and/or no microplastics and/or no formaldehyde, paraformaldehyde and formaldehyde releaser, and/or no diazolidinyl urea and/or no furfuryl alcohol and/or no iodopropynyl butylcarbamate, and/or no 2-bromo-2-nitropropane-1,3-diol and/or no 5-bromo-5-nitro-1,3-dioxane and other halogen-organic preservatives and/or other halogenated organic compounds, and/or no triclosan, and/or no polycyclic moschus compounds.

The powdery composition according to the invention comprises at least one substrate which is not a salt. This means, that the substrate is neutral and does not contain any charges in form of cations and anions. This has the advantage that the at least one substrate contributes to the desired viscosity of the aqueous cleansing composition, once the at least one substrate is dissolved in water in order to obtain the aqueous cleansing composition according to the invention. The at least one substrate acts as carrier within the powdery composition according to the invention. This has the further advantage that components which are liquid under normal conditions can be up-taken onto the at least one substrate and thus transferred into powder form. Thus, it is possible to include liquid components into the powdery composition according to the invention, wherein, however, such liquid components are then not liquid anymore, since they are up-taken onto the at least one substrate and thus transferred into powder form. Thus, a powdery composition according to the invention can be obtained, wherein the components are all in powder form.

The at least one substrate is selected from lactose, silica, mannite, xylite, diatomite, cellulose and derivates thereof, monocrystalline cellulose, modified cellulose, trimethyl glycine, saccharose, monosaccharide, polysaccharide, cyclodextrin, native starch, chemically modified starch, thermal modified starch, sorbitol, polyvinylpyrrolidone, silicate. Suitable derivates of cellulose are water soluble cellulose ether, e.g. methylhydroxy ethyl cellulose (MHEC), ethyl cellulose, hydroxyethyl cellulose (HEC). A suitable silica is for example known under the tradename Aerosil^{®} 200. A suitable diatomite is for example known under the trade name Celite^{®}. Suitable modified starches are e.g. distarch phosphate, monostarch phosphate, natural starches like corn starch, tapioca starch, potatoe starch. A suitable polyvinylpyrrolidone is known under the trade name Kollidon^{®}. Suitable silicates are for example kaolin, bentonite, or known under the trade name Veegum^{®}. Suitable polysaccharides are for example guar gum, gum arabic, alginic acid, galactomannan, talha gum. Suitable mono- or polysaccharids are for example glucose, maltodextrin, inositol, xylitol or dextrin. Preferred are sorbitol, maltodextrin, dextrin, mannite, xylite or mixtures thereof.

The amount of the at least one substrate present in the powdery composition according to the invention is at least 20 g, or at least 21 g, or at least 22 g, or at least 23 g, or at least 24 g, or at least 25 g, or at least 27 g, or at least 30 g, or at least 32 g, or at least 35 g, or at least 37 g and at most 55 g, or at most 54 g, or at most 53 g, or at most 52 g, or at most 51 g, or at most 50 g, or at most 47 g, or at most 45 g, or at most 42 g, or at most 40 g, wherein the amount of the at least one substrate present in the powdery composition according to the invention is based on 100 g of the total powdery composition. Suitable weight ranges of the at least one substrate present in the powdery composition according to the invention are at least 20 g and at most 55 g, or at least 25 g and at most 50 g, or at least 30 g and at most 45 g, wherein the amount of the at least one substrate present in the powdery composition according to the invention is based on 100 g of the total powdery composition.

The powdery composition according to the invention further comprises at least one viscosity adjusting agent. This has the advantage that the aqueous cleansing composition made from the powdery composition according to the invention when water is added to the powdery composition has a viscosity which is comparable to the viscosity of commercially available gel-like, ready-to-use aqueous cleansing compositions. The at least one viscosity adjusting agent present in the powdery composition according to the invention can be either selected from thickeners, i.e. from compounds thickening the water or from rheology modifier, i.e. compounds inducing the thickening of other compounds present in the powdery composition according to the invention. However, either way results in an aqueous composition which is not liquid anymore but has a viscosity in a desired range. The at least one viscosity adjusting agent present in the powdery composition according to the invention is selected from natural polymers, in particular natural thickener or gum, synthetic thickeners, ethers, alkoxylated alcohols, hydrophilic colloids or derivatives thereof, salts of mono- or divalent cations or mixtures thereof. Examples for suitable natural polymers are polysaccharides like xanthan gum, algin, carrageenan, gellan gum, acacia senegal gum, cellulose gum, modified potato starch, agarose, caesalpinia spinosa gum, cyamopsis tetragonoloba (guar) gum, aluminum starch octenylsuccinate, zea mays (corn) starch, dextrin, pectin, hyaluronic acid, glucomannan, hydrophilic colloids and derivatives thereof are cetyl hydroxyethyl cellulose, hydroxypropyl methyl cellulose, hydroxypropyl guar, hydroxyethyl cellulose, methyl methacrylate crosspolymer, carboxymethyl hydroxypropyl guar and silica, natto gum, sodium carboxymethyl starch, or mixtures thereof. Suitable examples for synthetic thickeners are synthetic polymers like carbomer, acrylates, C10 to C30 acrylate crosspolymer, sodium acrylate crosspolymer-2, sodium magnesium silicate, PVP. A suitable example for starch-ether based ingredients is hydroxypropyl starch phosphate, for alkoxylated alcohols is PEG-120 methyl glucose dioleate or PEG-18 glyceryl oleate. Examples for suitable salts of monovalent or divalent cations are alkali metal salts, alkaline earth metal salts and/or an ammonium salts and are, in particular, inorganic salts like dead sea salt, sodium chloride, magnesium chloride, potassium chloride, calcium chloride, ammonium chloride or mixtures thereof. Preferred viscosity adjusting agents are sodium chloride, potassium chloride, ammonium chloride or mixtures thereof.

The amount of the at least one viscosity adjusting agent optionally present in the powdery composition according to the invention is at least 0.01 g, or at least 0.03 g, or at least 0.05 g, or at least 0.07 g, or at least 0.1 g, or at least 0.15 g, or at least 0.2 g, or at least 0.5 g, or at least 1 g, or at least 1.5 g, or at least 2 g, or at least 2.5 g, or at least 3 g, or at least 3.5 g, or at least 4 g, or at least 5 g and at most 13 g, or at most 12 g, or at most 11 g, or at most 10 g, or at most 9.5 g, or at most 9 g, or at most 8.5 g, or at most 8 g, or at most 7.5 g, or at most 7 g, or at most 5 g, wherein the amount of the at least one viscosity adjusting agent present in the powdery composition according to the invention is based on 100 g of the total powdery composition. Suitable weight ranges of the at least one viscosity adjusting agent present in the powdery composition according to the invention are at least 0.01 g and at most 13 g, or at least 3 g and at most 10 g, or at least 5 g and at most 8 g, wherein the amount of the at least one viscosity adjusting agent present in the powdery composition according to the invention is based on 100 g of the total powdery composition.

The powdery composition according to the invention further comprises at least one surfactant. The at least one surfactant has the advantage to provide the cleansing, in particular the hair and skin cleansing properties to the aqueous cleansing composition according to the invention, once the at least one surfactant comprised in the powdery composition according to the invention is mixed with water and dissolved therein. In addition, the at least one surfactant provides the foaming properties to the aqueous cleansing composition according to the invention.

The at least one surfactant can be selected from one or more anionic surfactants, one or more amphoteric surfactants, one or more non-ionic surfactants or mixtures thereof. Suitable anionic surfactants are selected from isethionate, amino acid, sulfonic acid, sulfuric acid ester, alkyl sulfate, alkylether sulfate, taurate, sulfosuccinate, sulfosuccinamate, carboxylate, or mixtures thereof. Examples of suitable isoethionate are sodium cocoyl isethionate, sodium lauroyl methyl isethionate. Examples for suitable amino acids are sodium cocoyl glutamate, sodium cocoyl glycinate, sodium lauryl glycinate. Examples for suitable sulfonic acids are sodium lauryl sulfoacetate. Examples for suitable sulfuric acid esters are sodium C₁₄ to C₁₆ olefin sulfonate. An example for carboxylate is sodium lauryl glucose carboxylate. Examples for alkyl sulfates or alkylether sulfates are sodium lauryl sulfate, sodium laureth sulfate, sodium coco-sulfate, ammonium lauryl sulfate, ammonium laureth sulfate. Examples for suitable taurates are sodium methyl oleoyl taurate, sodium methyl cocoyl taurate, sodium lauroyl methyl taurate. Examples of suitable sulfosuccinate or sulfosuccinamate are disodium laureth sulfosuccinate, disodium lauryl sulfosuccinate. Preferably, the at least one surfactant is no ethoxylated surfactant. It is particularly suitable to use a combination of two or more surfactants in the powdery composition according to the invention. Preferred anionic surfactants are sodium coco-sulfate, sodium methyl oleoyl taurate, disodium lauryl sulfosuccinate, sodium lauryl sulfoacetate, sodium cocoyl glutamate, disodium lauryl sulfosuccinate, sodium lauryl sulfate, ammonium lauryl sulfate and mixtures thereof. Suitable non-ionic surfactants are amides and/or alkanol amides, glucosides (like lauryl glucoside). Examples for suitable amides or alkanolamides are lauramide MEA, lauramide MIPA, cocamide methyl MEA. Suitable amphoteric surfactants are sodium cocoamphoacetate, cocamidopropyl betaine.

The amount of the at least one surfactant present in the powdery composition according to the invention is at most 70 g, or at most 67 g, or at most 65 g, or at most 63 g, or at most 60 g, or at most 57 g, or at most 55 g, or at most 53 g, or at most 51 g, or at most 50 g, or at most 47 g, or at most 45 g, or at most 43 g, or at most 40 g and at least 15 g, or at least 17 g, or at least 20 g, or at least 23 g, or at least 25 g, or at least 27 g, or at least 30 g, or at least 33 g, or at least 35 g, wherein the amount of the at least one surfactant present in the powdery composition according to the invention is based on 100 g of the total powdery composition. Suitable weight ranges of the at least one surfactant present in the powdery composition according to the invention are at least 15 g and at most 70 g, or at least 20 g and at most 60 g, or at least 25 g and at most 47 g, wherein the amount of the at least one surfactant present in the powdery composition according to the invention is based on 100 g of the total powdery composition.

The powdery composition according to the invention optionally further comprises at least one chelating agent. This has the advantage that independently of the water used for mixing with the powdery composition according to the invention in order to obtain the aqueous cleansing composition according to the invention, said aqueous cleansing composition will have the desired viscosity and that the components of the powdery composition readily dissolve in the water. This is achieved since the at least one chelating agent chelates e.g. calcium, magnesium and other transition metals contained in the water which is added to the powdery composition in order to obtain the aqueous cleansing composition according to the invention. In addition, the at least one chelating agent helps to stabilize the obtained gel-like, ready-to-use aqueous cleansing composition, and in case of coloured aqueous cleansing compositions aids at avoiding discoloration, boosting performance of antioxidants if present, and/or boosting performance of preservatives (if present). The at least one chelating agent is selected from gluconate, citrate, polyaspartic acid, imino disuccinate, ethylendiamine disuccinate, polyphosphate or salts thereof, modified polyamine, sodium diethylenetriamine pentamethylene phosphonate, sodium glucoheptanoate, sodium gluconate, sodium phytate, tetrasodium glutamate diacetate (Na₄-GLDA), trisodium ethylenediamine disuccinate (Na₃-EDDS), sodium polyphosphate. Preferred chelating agents are sodium polyphosphate, citrate, tetra sodium GLDA, tetra sodium ethylenediamine disuccinate or mixtures thereof. Preferably, the at least one chelating agent is not EDTA.

The amount of the at least one chelating agent present in the powdery composition according to the invention is at least 0 g, or at least 0.01 g, or at least 0.03 g, or at least 0.05 g, or at least 0.07 g, or at least 0.1 g, or at least 0.15 g, or at least 0.2 g, or at least 0.5 g, or at least 1 g, or at least 1.5 g, or at least 2 g, or at least 2.5 g, or at least 3 g, or at least 3.5 g, or at least 4 g, or at least 5 g and at most 13 g, or at most 12 g, or at most 11 g, or at most 10 g, or at most 9.5 g, or at most 9 g, or at most 8.5 g, or at most 8 g, or at most 7 g, or at most 5 g, wherein the amount of the at least one chelating agent present in the powdery composition according to the invention is based on 100 g of the total powdery composition. Suitable weight ranges of the at least one chelating agent present in the powdery composition according to the invention are at least 0.01 g and at most 13 g, or at least 1 g and at most 10 g, or at least 1.5 g and at most 5 g, wherein the amount of the at least one chelating agent present in the powdery composition according to the invention is based on 100 g of the total powdery composition.

The powdery composition according to the invention optionally further comprises at least one fragrance and/or at least one essential oil. This has the advantage that the powdery composition according to the invention as well as the aqueous cleansing composition made therefrom when water is added to the powdery composition has a pleasant smell. The at least one fragrance and/or the at least one essential oil can be used in liquid form (e.g. as liquid on the basis of isopropyl myristate (IPM) or dipropylene glycol) or in powderous form (i.e. on the basis of maltodextrine). The at least one fragrance optionally present in the powdery composition according to the invention is commercially available from Givaudan Fragrances, Mane Deutschland GmbH or Düllberg Konzentra GmbH. The at least one or more essential oil optionally present in the powdery composition according to the invention can be selected from e.g. rosemary oil, orange oil, citrus oil, lime oil, lemongras oil, lavender oil, sandalwood oil, amyris oil, geranium oil, palmarosa oil, cedernwood oil, juniper oil, rosmary oil, ylang ylang oil, or mixtures thereof or any other suitable essential oil commonly used.

The amount of the at least one fragrance and/or essential oil optionally present in the powdery composition according to the invention is at least 0.0 g, or at least 0.01 g, or at least 0.03 g, or at least 0.05 g, or at least 0.07 g, or at least 0.1 g, or at least 0.15 g, or at least 0.2 g, or at least 0.5 g, or at least 1 g, or at least 1.5 g, or at least 2 g, or at least 2.5 g, or at least 3 g, or at least 3.5 g, or at least 4 g, or at least 5 g and at most 13 g, or at most 12 g, or at most 11 g, or at most 10 g, or at most 9.5 g, or at most 9 g, or at most 8.5 g, or at most 8 g, or at most 7 g, or at most 5 g, wherein the amount of the at least one fragrance present in the powdery composition according to the invention is based on 100 g of the total powdery composition. Suitable weight ranges of the at least one fragrance present in the powdery composition according to the invention are at least 0.01 g and at most 13 g, or at least 1 g and at most 10 g, or at least 1.5 g and at most 5 g, wherein the amount of the at least one fragrance present in the powdery composition according to the invention is based on 100 g of the total powdery composition.

The powdery composition according to the invention optionally further comprises at least one dye. This has the advantage that the powdery composition according to the invention as well as the aqueous cleansing composition made therefrom when water is added to the powdery composition has a colored appearance. The at least one dye optionally present in the powdery composition according to the invention is selected from synthetic dyes, natural dyes or dyeing extracts or mixtures thereof. Suitable dyes are for example dyes of the following color index numbers (C.I. numbers) 10020, 10316, 13015, 14700, 14720, 14815, 15510, 15980, 15985, 16035, 16185, 16230, 16255, 16290, 17200, 18050, 18130, 18690, 18736, 18820, 18965, 19140, 20470, 21230, 24790, 27755, 28440, 40215, 42045, 42051, 42053, 42080, 42090, 42100, 42170, 42510, 42520, 42735, 44045, 44090, 45100, 45190, 45220, 45350, 45370, 45380, 45396, 45405, 45410, 45430, 47005, 50325, , 50420, 59040, 60724, 60730, 61570, 61585, 62045, 74180, 75470, 75810, or mixtures thereof. Further suitable dyes are for example selected from caramel, capsanthin, capsorubin, beetroot red, anthocyanins, gardenia jasminoides fruit extract, hydrolyzed gardenia florida extract, beta vulgaris root juice, brassica oleracea capitata leaf extract, ribes nigrum callus powder, spirulina platensis powder, vaccinium myrtillus fruit powder, vaccinium macrocarbon fruit powder, citrus reticula peel powder, ipomoea batates tuber extract, hippophae rhamnoides fruit extract, carthamus tinctorius flower powder, pyrus malus fruit extract, malpighia glabra fruit extract, astaxanthin, haematococcus pluvialis extract, rubus fruticosus fruit extract, citrus aurantium dulcis fruit powder, fragaria ananassa fruit powder, ribes rubrum callus powder, rubus ideaus callus powder, daucus carota sativa root extract, raphanus sativus root extract, or mixtures thereof. Preferred dyes are C.I. 14700, 42090, 45350, 75810, gardenia jasminoides fruit extract, spirulina platensis powder, beta vulgaris juice, or mixtures thereof.

The amount of the at least one dye optionally present in the powdery composition according to the invention is at least 0.0 g, or at least 0.0001 g, or at least 0.005 g, or at least 0.001 g, or at least 0.005g, or at least 0.01 g, or at least 0.03 g, or at least 0.05 g, or at least 0.07 g, or at least 0.1 g, or at least 0.15 g, or at least 0.2 g, or at least 0.5 g, or at most 1 g, or at most 1.5 g, or at most 2 g, or at most 2.5 g, or at most 3 g, or at most 3.5 g, or at most 4 g, or at most 5 g and at most 13 g, or at most 12 g, or at most 11 g, or at most 10 g, or at most 9.5 g, or at most 9 g, or at most 8.5 g, or at most 8 g, or at most 7 g, or at most 5 g, wherein the amount of the at least one dye present in the powdery composition according to the invention is based on 100 g of the total powdery composition. Suitable weight ranges of the at least one dye present in the powdery composition according to the invention are at least 0,0001 g and at most 13 g, or at least 0,0005 g and at most 10 g, or at least 0,001 g and at most 5 g, wherein the amount of the at least one dye present in the powdery composition according to the invention is based on 100 g of the total powdery composition.

The powdery composition according to the invention optionally further comprises at least one preserving agent. This has the advantage that the powdery composition according to the invention as well as the aqueous cleansing composition made therefrom when water is added to the powdery composition stay microbiological stable and usable. In particular, assuming that impure water or an unclean vessel is used for making or storing the aqueous cleansing composition according to the invention, there is a certain risk of e.g. bacteria growth in the aqueous cleansing composition which then becomes unusable. The presence of at least one preserving agent, however, stabilizes microbiologically the aqueous cleansing composition and keeps the aqueous composition usable. The at least one preserving agent optionally present in the powdery composition according to the invention is selected from organic acids, halogenorganic compounds, amides, quaternary ammonium compounds, esters, alcohols, carbonic acids and derivatives thereof, thio-compounds, phenols and derivatives thereof, amines or mixtures thereof. Examples for organic acids are benzoic acid or salts thereof, sorbinic acids or salts thereof, salicylic acids or salts thereof. Example for halogen organic compounds is climbazole. Examples for amides are imidazolidinyl urea, diazolidinyl urea, hexamidine diisethionate, triclocarban, piroctone olamine, chloroactamide, iodopropynyl butylcarbamate. Examples for quaternary ammonium compounds are benzethonium chloride, cetrimonium bromide, cetrimonium chloride, behentrimonium chloride. Examples for esters are butyl paraben, isobutyl paraben, methyl paraben, propyl paraben, sodium ethylparaben. Suitable alcohols are chloro phenesin, chloro butanol, 2-bromo-2-nitropropane-1,3-diol. An example for thio-compounds is zinc pyrithione. Examples for phenols are chloroxylenol, triclosan, o-cymen-5-ol. An example for phenol ether is phenoxyethanol. An example for amines is methenamine. It is particularly suitable to use a combination of two or more preserving agents in the powdery composition according to the invention. Preferred preserving agents are organic acids like sodium benzoate, potassium sorbate, dehydroacetic acid, benzyl alcohol, sodium anisate, caprylhydroxamic acid, phenethyl alcohol, sodium levulinate, or mixtures thereof.

The amount of the at least one preserving agent optionally present in the powdery composition according to the invention is at least 0.0 g, or at least 0.1 g, or at least 0.2 g, or at least 0.5 g, or at least 1 g, or at least 1.5 g, or at least 2 g, or at least 2.5 g, or at least 3 g, or at least 3.5 g, or at least 4 g, or at least 5 g, or at least 6 g, or at least 7 g and at most 12 g, or at most 10 g, or at most 9 g, or at most 8 g, wherein the amount of the at least one preserving agent present in the powdery composition according to the invention is based on 100 g of the total powdery composition. Suitable weight ranges of the at least one preserving agent present in the powdery composition according to the invention are at least 0.1 g and at most 12 g, or at least 0.5 g and at most 10 g, or at least 1 g and at most 5 g, wherein the amount of the at least one preserving agent present in the powdery composition according to the invention is based on 100 g of the total powdery composition.

The powdery composition according to the invention optionally further comprises at least one pH adjusting agent. This has the advantage that the aqueous cleansing composition made from the powdery composition according to the invention when water is added to the powdery composition has an eudermic pH. The at least one pH adjusting agent optionally present in the powdery composition according to the invention is selected from organic acids or salts thereof. Suitable examples for organic acids are citric acid, or salts thereof like sodium citrate, trisodium citrate, tricalcium citrate; phytic acid or salts thereof; glycolic acid or salts thereof; ascorbic acid or salts thereof; maltobionic acid or salts thereof; ammonium acetate; apple vinegar; adipic acid or salts thereof like sodium adipate, lactic acid or salts thereof like sodium lactate; lacobiotic acid; malic acids or salts thereof like sodium malate; tatric acid or salts thereof like sodium tartrate. Preferred pH adjusting agents are citric acid, sodium citrate, lactic acid, sodium lactate, or mixtures thereof.

The amount of the at least one pH adjusting agent optionally present in the powdery composition according to the invention is at least 0.0 g, or at least 0.01 g, or at least 0.03 g, or at least 0.05 g, or at least 0.07 g, or at least 0.1 g, or at least 0.15 g, or at least 0.2 g, or at least 0.5 g, or at least 1 g, or at least 1.5 g, or at least 2 g, or at least 2.5 g, or at least 3 g, or at least 3.5 g, or at least 4 g, or at least 5 g and at most 13 g, or at most 12 g, or at most 11 g, or at most 10 g, or at most 9.5 g, or at most 9 g, or at most 8.5 g, or at most 8 g, or at most 7 g, or at most 5 g, wherein the amount of the at least one pH adjusting agent present in the powdery composition according to the invention is based on 100 g of the total powdery composition. Suitable weight ranges of the at least one pH adjusting agent present in the powdery composition according to the invention are at least 0.01 g and at most 13 g, or at least 1 g and at most 10 g, or at least 1.5 g and at most 7 g, wherein the amount of the at least one pH adjusting agent present in the powdery composition according to the invention is based on 100 g of the total powdery composition.

The powdery composition according to the invention optionally further comprises at least one active ingredient. This has the advantage that the aqueous cleansing composition made from the powdery composition according to the invention when water is added to the powdery composition provides nutritive substances to hair and/or skin which strengthens hair/skin, provides anti-ageing properties, anti-acne properties, anti-pollution properties, firmness, elasticity, metabolic activation, microbiota modulation, moisturizing properties, peeling/skin renewal properties, soothe/protection. The at least one active ingredient is selected from plant extracts e.g. aloe vera, green tea, matcha, acai, grape seed, oat, mallow, hibiscus, walnut, arnica, chestnut, prickle pear, wheat bran, borage seed, line seed, olive, ginger, bamboo, hemp, coconut, frankincense, apple, lemon, saccharomyces ferment, bioferment, radish root ferment, algae and microalgae (e.g. spirulina), or any other suitable plant extracts commonly used, or mixtures thereof.

The amount of the at least one active ingredient optionally present in the powdery composition according to the invention is at least 0.0 g, or at least 0.01 g, or at least 0.03 g, or at least 0.05 g, or at least 0.07 g, or at least 0.1 g, or at least 0.15 g, or at least 0.2 g, or at least 0.5 g, or at least 1 g, or at least 1.5 g, or at least 2 g, or at least 2.5 g, or at least 3 g, or at least 3.5 g, or at least 4 g, or at least 5 g, or at least 6 g, or at least 7 g, or at least 8 g, or at least 9 g and at most 25 g, or at most 24 g, or at most 23 g, or at most 22 g, or at most 21 g, or at most 20 g, or at most 18 g, or at most 16 g, or at most 14 g, or at most 12 g, wherein the amount of the at least one active ingredient present in the powdery composition according to the invention is based on 100 g of the total powdery composition. Suitable weight ranges of the at least one active ingredient present in the powdery composition according to the invention are at least 0.01 g and at most 25 g, or at least 1.5 g and at most 20 g, or at least 2 g and at most 15 g, wherein the amount of the at least one active ingredient present in the powdery composition according to the invention is based on 100 g of the total powdery composition.

The powdery composition according to the invention optionally further comprises at least one conditioning agent. This has the advantage that the aqueous cleansing composition made from the powdery composition according to the invention when water is added to the powdery composition provides nutritive substances to hair and/or skin which makes the skin and/or hair soft, healthy, protect from drying out skin/hair, anti-fizzing and anti-static hair, improves compatibility of wet and dry hair, shining hair, humectant/moisturizers, strengthen skin. The at least one conditioning agent optionally present in the powdery composition according to the invention is selected from amides, amines, urea compounds, esters, vitamins, amino acid derivates, hydrolyzed proteins, hydrolyzed collagen, quaternary ammonium compounds, plant-based oils or plant-based butters or mixtures thereof. Preferably, a combination of two or more conditioning agents are present in the powdery composition. Suitable examples for amides are allantoin and urea. Suitable examples for amines are stearamidopropyl dimethyl amine, brassicamidopropyl dimethyl amine, sphinganine, diaminopyrimidine oxide. Suitable urea compound is 1-methylhydantoin-2-imide. Suitable ester is acetylphytosphingosine. Suitable examples for vitamins are nicotinic acid amide, ubiquinon and panthenol. Suitable examples for amino acid derivates are sodium PCA, sodium polyasparate and zinc PCA. Suitable examples for hydrolyzed proteins are hydrolyzed wheat protein, sodium cocoyl hydrolyzed rice protein, hydrolyzed corn protein and hydrolyzed oat protein. Suitable examples for quaternary ammonium compounds are natural betain as well as cetrimonium chloride, behentrimonium chloride, stearalkonium chloride. Suitable examples for plant-based oils are avocado oil, sesame oil, jojoba oil, sunflower oil, soy oil, baobab oil, marula oil, pomegranate seed oil, hemp oil. Suitable examples for plant-based butters are cocoa butter, shea butter, coconut oil, shorea butter. Preferred conditioning agents are arginine, guar hydroxypropyltrimonium chloride, sweet almond oil, natural betain, or mixtures thereof.

The amount of the at least one conditioning agent optionally present in the powdery composition according to the invention is at least 0.0 g, or at least 0.01 g, or at least 0.03 g, or at least 0.05 g, or at least 0.07 g, or at least 0.1 g, or at least 0.15 g, or at least 0.2 g, or at least 0.5 g, or at least 1 g, or at least 1.5 g, or at least 2 g, or at least 2.5 g, or at least 3 g, or at least 3.5 g, or at least 4 g, or at least 5 g, or at least 6 g, or at least 7 g, or at least 8 g, or at least 9 g and at most 25 g, or at most 24 g, or at most 23 g, or at most 22 g, or at most 21 g, or at most 20 g, or at most 18 g, or at most 16 g, or at most 14 g, or at most 12 g, wherein the amount of the at least one conditioning agent present in the powdery composition according to the invention is based on 100 g of the total powdery composition. Suitable weight ranges of the at least one conditioning agent present in the powdery composition according to the invention are at least 0.01 g and at most 25 g, or at least 1.5 g and at most 20 g, or at least 2 g and at most 15 g, wherein the amount of the at least one conditioning agent present in the powdery composition according to the invention is based on 100 g of the total powdery composition.

The powdery composition according to the invention optionally further comprises at least one moisturizing agent. This has the advantage that the aqueous cleansing composition made from the powdery composition according to the invention when water is added to the powdery composition provides moisture to the skin and/or hair. The at least one moisturizing agent optionally present in the powdery composition according to the invention is selected from glycosamino glycanes, urea compounds, amides, polysaccharides, amino acids or derivatives thereof, proteins, alcohols, plants, plant based oils and/or extracts, or mixtures thereof. Suitable examples for urea compounds are urea, niacinamid, betain, allantoin, calcium PCA, tetrahydro piperidine, pyridoxine HCI, caffeine, ectoin. A suitable example for an amide is panthenol. Suitable examples for polysaccharides are hyaluronic acid or salts thereof, PCA or salts thereof, xylitylglucoside, anhydro xylitol, xylitol, inulin, trehalose, sucrose cocoate, fucose. Suitable examples for amino acids or derivatives thereof are L-arginine, glutamine, polyglucamic acid, lysine, aminobutyric acid, sodium polyaspartate, acetylglutaminoyl farnesylcysteine, creatine, acetyl glycyl beta-alanine. Suitable examples for proteins are collagen, hydrolyzed elastin, hydrolyzed collagen, hydrolyzed keratin. Suitable examples for alcohols are bisabolol, hydroxyethyl urea or 1,2-hexanediol. A suitable plant is aloe vera, or aloe barbadensis leaf juice powder. Suitable examples for plant oils are sweet almond oil, jojoba oil, macadamia oil, avocado oil, mango butter, shea butter, olive oil, rapeseed oil, cocoa butter, coconut oil, peach kernel oil, hemp oil, argan oil or hydrogenated argania spinosa kernel oil, sunflower oil, punica granatum seed oil, brassica oleracea seed oil, garcinia indica seed butter, peanut oil, safflower seed oil, walnut seed oil, papaver somniferum seed oil, euterpe oleracea fruit oil, shorea robusta seed butter, pentaclethra macroloba seed oil, limnanthes alba (Meadowfoam) seed oil, tomato oil, nigella sativa seed oil, camellia japonica seed oil, castor seed oil, pumpkin seed oil, orbignya oleifera seed oil, adansonia digitata seed oil, hydrogenated pistacia vera seed oil, amaranthus caudatus seed oil, cranberry seed oil, santalum spicatum seed oil, sunflower oil, watermelon oil, rosa moschata seed oil, black currant oil, linseed oil, borago officinalis seed oil, cotton oil, sesame seed oil, bertholletia excelsa seed oil, theobroma grandiflorum seed butter, melia azadirachta seed oil, perilla ocymoides seed oil, opuntia ficus-indica seed oil, raspberry oil, wheat germ oil, salvia hispanica seed oil, bassia latifolia seed butter, astrocaryum vulgare fruit oil, passiflora edulis seed oil, isatis tinctoria seed oil, crambe abyssinica seed oil. A suitable example for glycosaminoglycanes is hyaluronic acid. Preferred moisturizing agents are selected from sweet almond oil, jojoba oil, avocado oil, mango butter, olive oil, hemp oil, coconut oil, argan oil, punica granatum seed oil, brassica oleracea seed oil, camellia japonica seed oil, amaranthus caudatus seed oil, or mixtures thereof.

The amount of the at least one moisturizing agent optionally present in the powdery composition according to the invention is at least 0.0 g, or at least 0.05 g, or at least 0.07 g, or at least 0.1 g, or at least 0.15 g, or at least 0.2 g, or at least 0.25 g, or at least 0.5 g, or at least 1 g, or at least 1.5 g, or at least 2 g, or at least 2.5 g, or at least 3 g, or at least 3.5 g, or at least 4 g, or at least 5 g and at most 13 g, or at most 12 g, or at most 11 g, or at most 10 g, or at most 9.5 g, or at most 9 g, or at most 8.5 g, or at most 8 g, or at most 7 g, or at most 5 g, wherein the amount of the at least one moisturizing agent present in the powdery composition according to the invention is based on 100 g of the total powdery composition. Suitable weight ranges of the at least one moisturizing agent present in the powdery composition according to the invention are at least 0.01 g and at most 13 g, or at least 0.2 g and at most 10 g, or at least 0.25 g and at most 5 g, wherein the amount of the at least one moisturizing agent present in the powdery composition according to the invention is based on 100 g of the total powdery composition.

The powdery composition according to the invention optionally further comprises at least one pearlescent agent and/or at least opacifying agent. This has the advantage that the aqueous cleansing composition made from the powdery composition according to the invention when water is added to the powdery composition has a pleasant appearance. The at least one pearlescent agent optionally present in the powdery composition according to the invention is selected from glycol distearate, glycol stearate; pigments like synthetic fluorphlogopite (e.g. synthetic mica, natural mica, calcium aluminum borosilicate), titanium dioxide, tin oxide, iron oxides, ferric ferrocyanide, caesalpinia sappan bark extract, indigofera tinctoria leaf extract; stearamidoethyl diethylamine; cetearyl alcohol; coco glucoside; polymethylsilsesquioxane; polyquaternium-7; PEG-7; glyceryl cocoate; methylsilanol; tri-PEG-8 glyceryl cocoate, styrene/acrylates copolymer, styrene/acrylamide copolymer; acrylates/methoxy PEG-10, maleate/styrene copolymer; or mixtures thereof.

The amount of the at least one pearlescent agent and/or at least opacifying agent optionally present in the powdery composition according to the invention is at least 0.0 g, or at least 0.1 g, or at least 0.15 g, or at least 0.2 g, or at least 0.5 g, or at least 1 g, or at least 1.5 g, or at least 2 g, or at least 2.5 g, or at least 3 g, or at least 3.5 g, or at least 4 g, or at least 5 g or at most 5 g, wherein the amount of the at least one pearlescent agent and/or at least opacifying agent present in the powdery composition according to the invention is based on 100 g of the total powdery composition. Suitable weight ranges of the at least one pearlescent agent and/or at least opacifying agent present in the powdery composition according to the invention are at least 0.01 g and at most 13 g, or at least 1 g and at most 10 g, or at least 1.5 g and at most 5 g, wherein the amount of the at least one pearlescent agent and/or at least opacifying agent present in the powdery composition according to the invention is based on 100 g of the total powdery composition.

The at least one powdery composition according to the invention optionally further comprises at least one emulsifier, in particular at least one o/w emulsifier. This has the advantage that the aqueous cleansing composition made from the powdery composition according to the invention when water is added to the powdery composition can be designed as shower creme, provides gloss and moisture. The at least one emulsifier can be selected from stearin, palmitic acid, glyceryl esters (e.g. polyglyceryl-10 laurate, polyglyceryl-3 cocoate, glyceryl stearate), potassium cocoate, potassium oleate, lecithin, hydrogenated lecithin, lysolecithin, or mixtures thereof. Preferred emulsifiers are selected from polyglyceryl-10 laurate, lecithin, hydrogenated lecithin, lysolecithin, or mixtures thereof.

The powdery composition according to the invention optionally further comprises at least one antioxidant. This has the advantage that the aqueous cleansing composition made from the powdery composition according to the invention when water is added to the powdery composition as well as the powdery composition are stabilized, and decomposition of the components contained is prevented. The at least one antioxidant optionally present in the powdery composition according to the invention is selected from tocopherol, tocopheryl acetate, ascorbic acid, ascorbyl glucoside, sodium or magnesium ascorbyl phosphate, BHT, BAH, quercetin, morin, 3,4-dihydroxybenzoic acid, thymol, carvacrol, ferulic acid, sinapic acid, caffeic acid, coumaric acid, rosmarinic acid, gallic acid, chlorogenic acid, rosmarinus officinalis (rosemary) leaf extract, salvia triloba leaf extract, ergothioneine, hydroxyacetophenone, green tea extract, or mixtures thereof. Preferred antioxidants are selected from tocopherol, tocopheryl acetate, ascorbic acid, ascorbyl glucoside, salvia triloba leaf extract, hydroxyacetophenone, green tea extract, rosmarinic acid, or mixtures thereof.

The amount of the at least one antioxidant optionally present in the powdery composition according to the invention is at least 0.0 g, or at least at least 0.05 g, or at least 0.07 g, or at least 0.1 g, or at least 0.15 g, or at least 0.2 g, or at least 0.5 g, or at least 1 g, or at least 1.5 g, or at least 2 g, or at most 2.5 g, or at most 3 g, or at most 3.5, or at most 4 g, or at most 5 g wherein the amount of the at least one antioxidant present in the powdery composition according to the invention is based on 100 g of the total powdery composition. Suitable weight ranges of the at least one antioxidant present in the powdery composition according to the invention are at least 0.05 g and at most 5 g, or at least 0.07 g and at most 4 g, or at most 0.1 g and at most 2 g, wherein the amount of the at least one antioxidant present in the powdery composition according to the invention is based on 100 g of the total powdery composition.

The at least one powdery composition according to the invention optionally further comprises at least one absorbent. This has the advantage that the pourability and flowability of the powdery composition according to the invention is maintained. The at least one absorbent can be selected from silica, hydrated silica, (modified) corn starch, aluminum starch octenylsuccinate, zea mays (corn) starch, magnesium aluminium silicate, charcoal powder, kaolin, cellulose, illite, talc, magnesium hydroxide, magnesium carbonate hydroxide or mixtures thereof. Preferred absorbents are selected from silica, hydrated silica, magnesium hydroxide, magnesium carbonate hydroxide, zea mays (corn) starch, or mixtures thereof.

The amount of the at least one absorbent optionally present in the powdery composition according to the invention is at least 0.0 g, or at least 0.01 g, or at least 0.03 g, or at least 0.05 g, or at least 0.1 g, or at least 0.15 g, or at least 0.2 g, or at least 0.3 g, or at least 0.4 g, or at least 0.5 g, or at least 1 g, or at least 1.5 g, or at least 2.0 g, or at least 2.5 g, or at least 3 g, or at most 8 g, or at most 7 g, or at most 6 g or at most 5.5 g or at most 5.0g, or at most 4.5 g, or at most 4.0 g wherein the amount of the at least one absorbent present in the powdery composition according to the invention is based on 100 g of the total powdery composition. Suitable ranges of the at least one absorbent present in powdery composition according to the invention are at least 0.01 g to at most 8 g, or at least 1 g to at most 6 g, or at least 3g to at most 5g, wherein the amount of the at least one absorbent present in the powdery composition according to the invention is based on 100 g of the total powdery composition.

Further, a method for manufacturing the powdery composition according to the invention is claimed, wherein the method comprises a mixing step, wherein in said mixing step all components in powder form of the powdery composition are mixed.

The components in powder form can be provided all at the same time to the mixing vessel. The components in powder form can also be provided one after the other and in any order to the mixing vessel. The components in powder form can also be provided batchwise to the mixing vessel, e.g. two components are provided into the mixing vessel, are mixed and then another two components are added and mixed.

In case the powdery composition according to the invention is designed as powder having free-flowing properties, the mixing step may already by designed such that the powdery composition according to the invention designed as powder having free-flowing properties is obtained.

It is possible to provide the components at once, or over a certain time period into the mixing vessel.

In case a liquid component should be present in the powdery composition according to the invention, this liquid component must first be transferred into a powder form. Consequently, the method for manufacturing the powdery composition according to the invention comprises an up-take step, wherein the at least one liquid component is up-taken onto the at least one substrate present in the powdery composition and thus transferred into a powder form. Thus, it is possible to manufacture a powdery composition in which all components are present in powder form. The up-take step is conducted prior to the mixing step. The up-take step can be conducted in the same vessel as the mixing step, or the up-take step can be conducted in a separate vessel. The order of addition and time of addition can vary as explained above with respect to the mixing step.

A suitable mixing vessel is every mixing vessel which allows mixing powdery and liquid components, like for example a Lödige mixer.

The time for mixing the components may vary between 10 seconds and up to 5 minutes. The mixing speed is dependent on the geometry of the mixing vessel and can be conducted at a speed of between 50 rpm up to 150 rpm. In case temperature sensitive components are present, care should be taken that during the mixing the temperature is maintained at a certain temperature level e.g. not more than 35 °C.

Optionally, after the mixing step, the method for manufacturing the powdery composition according to the invention may further comprise a step in which the powder obtained in the mixing step is subjected to an adhering and/or sticking step, in which the particles are adhered and/or sticked together in order to obtain a powdery composition according to the invention designed as powdery having no free-flowing properties. Such an adhering and/or sticking step may encompass physical (e.g. melting) and/or chemical (e.g. gluing) and/or mechanical means (e.g. pressing) or a combination thereof. For example the adhering and/or sticking step may be designed as pressing step in which the particles of the powder obtained in the mixing step are pressed together in order to form a pellet.

The present invention further comprises an aqueous cleansing composition, wherein the aqueous cleansing composition comprises the powdery composition according to the invention in dissolved form. The aqueous cleansing composition according to the invention has the advantage of having comparable or even improved properties like the commercially available, gel-like, ready-to-use cleansing compositions. E.g. the aqueous cleansing composition according to the invention has improved foaming properties, although the amount of surfactant present in the aqueous cleansing composition according to the invention is lower than in commercially available, gel-like, ready-to-use cleansing compositions. The amount of at least one surfactant present in the aqueous composition according to the invention is not more than 14 g, or not more than 13 g, or not more than 12 g, or not more than 11 g, or not more than 10 g, or not more than 8 g, or not more than 7 g, or not more than 6 g, or not more than 5 g based on 100 g of the total aqueous composition. In contrast thereto, the amount of surfactant present in commercially available, gel-like, ready-to-use cleansing compositions is usually at least 15 g based on 100g of the total commercially available, gel-like, ready-to-use cleansing composition. The amount of the at least one surfactant in the aqueous composition according to the invention is based on a weight ratio of powdery composition according to the invention : added water of 1:4. This means that for comparison reasons, the amount of the at least one surfactant present in the commercially available, gel-like, ready-to-use cleansing compositions is calculated on the same basis, i.e. the weight ratio of a (hypothetical) dry weight of all components present: (hypothetically added) water is 1:4. In case a different weight ratio of powdery composition according to the invention : added water is chosen, the maximum amount of the at least one surfactant present may be higher or lower than the above defined values. In such a case, for comparison reasons, the calculation has to be adapted for the commercially available cleansing compositions accordingly.

The pH of aqueous cleansing composition according to the invention is preferably not less than 4.0 and not more than 6.0, preferably not more than 4.1 to not more than 5.8. Preferably, the pH of the aqueous cleansing composition according to the invention is in the range of 4.5 to 5.0 in case it is designed as shower gel or soap or in the range of 4.9 to 5.4 in case it is designed as shampoo. However, if the aqueous cleansing composition according to the invention is designed as cleansing composition for animals, the pH range is between 5.0 and up to 7.5.

The aqueous cleansing composition according to the invention differs from the powdery composition according to the invention only in that the aqueous cleansing composition contains as additional component water. Due to the addition of water to the powdery composition according to the invention, the aqueous cleansing composition according to the invention is obtained, wherein the aqueous cleansing composition is designed as gel-like, ready-to-use cleansing composition. In particular, the aqueous cleansing composition is selected from the group consisting of shampoo, 2-in-1 shampoo conditioner, shower gel, shower cream, hair and body wash, liquid hand soaps, facial cleanser or combinations thereof for human beings or animals.

Thus, herewith claimed is also the use of the aqueous cleansing composition according to the invention as shampoo, 2-in-1 shampoo conditioner, shower gel, shower cream, hair and body wash, liquid hand soaps, facial cleanser or combinations thereof for human beings or animals.

The aqueous cleansing composition according to the invention has the advantage that it has a comparable viscosity, cleansing, and conditioning properties and equal or improved foaming properties compared to commercially available, gel-like, ready-to-use aqueous cleansing compositions, although the water is added only before use, e.g. at home or at the manufacturing site of a distributer, and not in the manufacturing site of the producer of the powdery composition like it is the case for commercially available, gel-like, ready-to-use aqueous cleansing compositions. In particular, the aqueous cleansing composition according to the invention has a viscosity in the range of at least 3000 mPa*s and up to 15000 mPa*s, preferably in the range of at least 4000 mPa*s and at most 9000 mPa*s. Commercially available, gel-like, ready-to-use aqueous cleansing compositions have a viscosity in the range of 2500 mPa*s and up to 15000 mPa*s. The viscosity is measured using a Brookfield viscosimeter at 20 +/-5°C, 0.5 spindle and 20 rpm.

Further, a method for manufacturing the aqueous cleansing composition is claimed, wherein the method comprises mixing the powdery composition according to the invention with water, wherein the water is selected from deionized water, purified water, mineral water or tap water, or a mixture thereof. It is a particular advantage of the powdery composition according to the invention that every water can be used for manufacturing the aqueous composition according to the invention, but the aqueous composition according to the invention has independently of the water used, identical properties in terms of e.g. viscosity, cleansing, conditioning, or foaming properties.

The mixing of water and the powdery composition according to the invention is done by shaking and/or stirring. After shaking and/or stirring it may be necessary to let the thus obtained aqueous composition according to the invention stand for some time (e.g. for 30 minutes up to 8 hours) to allow the components to be dissolved completely and to built-up viscosity.

The amount of water used for mixing with the powdery composition according to the invention may vary from (water: powdery composition) 2.5:1 to 4.5:1, or 3:1 to 4:1, i.e. may be at least 2.5:1, or at least 2.6:1, or at least 2.7:1, or at least 2.8:1, or at least 2.9:1, or at least 3.0:1, or at least 3.1:1, or at least 3.2:1, or at least 3.3:1, or at least 3.4:1, or at least 3.5:1, or at least 3.6:1, or at least 3.7:1, or at least 3.8:1, or at least 3.9:1, or at most 4.0:1, or at most 4.1:1, or at most 4.2:1, or at most 4.3:1, or at most 4.4:1, or at most 4.5:1. Particularly preferred is the ratio of water: powdery composition according to the invention of 4:1.

The temperature of the water added to the powdery composition according to the invention can vary between 15 °C and up to 45 °C. Temperatures of between 35 °C to 42°C are preferred.

In addition, the use of the powdery composition according to the invention for manufacturing an aqueous cleansing composition according to the invention, wherein the aqueous cleansing composition according to the invention comparable viscosity, cleansing, and conditioning properties and equal or improved foaming properties compared to commercially available, gel-like, ready-to-use aqueous cleansing compositions.

### EXAMPLES

The invention will be explained in the following with reference to particular examples. However, the invention should not be limited to these examples.

### Example 1: Manufacturing of a powdery composition according to the invention

Up-take step: In a Lödige mixer, 32,15 g of sorbitol (substrate which is no salt) are provided. Under stirring, 0.5 g of sweet almond oil (conditioning agent) and 1.0 g of a fragrance oil are added to the sorbitol. The mixing is conducted until all oil is up-taken onto the sorbitol, which was the case after 60 seconds stirring at 120 rpm.

Mixing step: the obtained powder (oils up-taken on sorbitol) is left in the mixer, and the following powdery components are added to the powder: 2.4 g arginine (conditioning agent), 0.8 g guar hydroxypropyltrimonium chloride (conditioning agent), 7,3 g sodium chloride (viscosity adjusting agent), 3.75 g citric acid (pH adjusting agent), 2.5 g sodium polyphosphate (chelating agent), 0.75 g sodium benzoate (preserving agent), 1.75 g potassium sorbate (preserving agent) 0.1 g aloe vera freeze dried powder (moisturizing agent). The powdery components are mixed for 50 seconds at 120 rpm. Finally, the following powdery components are added to the obtained powdery mixture: 28.0 g sodium coco sulfate (surfactant), 6.5 g sodium methyl oleoyl taurate (surfactant) and 12.5 g of disodium lauryl sulfosuccinate (surfactant). All powdery components are mixed for 20 seconds at 100 rpm, and 100 g of a powdery composition according to the invention is obtained.

The D50 particle size (median) of the thus obtained powdery composition was about 178 µm and the D90 particle size (median) was 549 µm (measured using a Mastersizer 2000 at 3 bar) and the residual moisture content was 1.1 % (measured according to DIN51718). The amount of surfactant present in the powdery composition was 47 g.

### Example 2: Manufacturing of an aqueous composition according to the invention

20 g of the powdery composition obtained in Example 1 were provided in a bottle. 80 g of tap water (40 °C) were added to the powdery composition. The bottle was closed and the content of the bottle was shaken by hand for 60 seconds. The powdery composition was completely dissolved in the water and no solid particles remained in the water. After the shaking was finished, a foam was observed on the surface. The content of the bottle was then allowed to stand for another (minimum) 30 minutes at room temperature. The foam decomposed within this time. An aqueous cleansing composition was obtained which had a yellowish, opalescent appearance. The measured viscosity was 8000 mPa*s (measured using a Brookfield viscosimeter at 20 +/-5 °C, 0.5 rod and 20 rpm). The amount of surfactant present in the aqueous cleansing composition was 9.4 g.

### Example 3: Examination of foaming properties of the aqueous composition according to the invention

The foaming properties of the aqueous composition according to the invention as obtained in Example 2 was examined and compared with the foaming properties of a commercially available, gel-like ready-to-use aqueous cleansing composition. 100 g of a commercially available, gel-like ready to use aqueous cleansing composition consisted of the following components: 80.0g water (purified), 1.30 g fragrance oil, 0.50 g PCA Glyceryl Oleate (conditioning agent), 0.20 g glyceryl oleate (conditioning agent), 0.30 g hydrated wheat protein (conditioning agent), 0.10 g allantoin (conditioning agent), 0.40 g sodium benzoate (preserving agent), 0.55 g citric acid (pH adjusting agent), 1.00 g sodium chloride (viscosity adjusting agent), 11.0 g sodium coco sulfate (surfactant), 0.50 g disodium cocoyl glutamate (surfactant), 3.00 g coco glucoside (surfactant) and 1,60 g cocamidopropyl betaine (surfactant). The viscosity of the commercially available aqueous cleansing composition was 12600 mPa/s. The amount of surfactant present in the commercially available aqueous cleansing composition was 16,1 g.

The examination of the foaming properties was made as follows: A 5 g sample of each aqueous cleansing composition was dissolved in 500 g of distilled water and heated to a temperature of 42 °C. 101 g of each thus obtained solution was carefully transferred into a 250ml beaker (Ø 70mm). No foam should be present in or on the solutions. The solutions were then stirred using a turbine stirrer (Ø 28mm). The stirrer is inserted into the solution only with half of its height (i.e. half of the stirrer was inside the solutions and the other half of the stirrer was outside of the solutions. The temperature of the solutions during this stirring must be kept in between 40 to 42 °C. The heights of the foam which is built on the surface of the solutions is measured using a regular ruler. The stirring was conducted for 120 seconds at 1500 rpm. A first series of measurements was made 30 seconds after the stirring was stopped. A second series of measurements was made 180 seconds after the stirring was stopped. A third series of measurements was made 300 seconds after the stirring was stopped. Each series of measurements consisted of four measurements, wherein each measurement was made at a different location. The results are summarized in the following table:

| | Height of the foam on the surface of the solutions [mm] | | | | | |
|---|---|---|---|---|---|---|
| Measurement | After 30 sec | | After 180 sec | | After 300 sec | |
| Solution | Cleansing composition (Ex. 2) | Comp. cleansing comp. | Cleansing composition (Ex. 2) | Comp. cleansing comp. | Cleansing composition (Ex. 2) | Comp. cleansing comp. |
| 1 | 47.0 | 47.0 | 45.0 | 45.0 | 44.5 | 44.0 |
| 2 | 48.0 | 46.0 | 46.0 | 44.0 | 46.0 | 43.0 |
| 3 | 48.0 | 45.0 | 46.0 | 44.0 | 45.5 | 42.0 |
| 4 | 46.5 | 47.0 | 45.0 | 45.0 | 44.0 | 44.0 |
| mean | 47.4 | 46.2 | 45.5 | 44.5 | 45.0 | 43.2 |

It is demonstrated that the powdery composition according to the invention results in a aqueous cleansing composition according to the invention has improved foam properties (height of the foam and stability of the foam) compared to a commercial available aqueous gel-like, ready-to-use cleansing composition although significantly less surfactant is used in the compositions according to the invention.

## Claims

1. Powdery composition, comprising
- at least one substrate, wherein the substrate is not a salt;
- at least one surfactant, wherein the amount of the at least one surfactant is at most 70 g, based on 100 g of the total powdery composition;
- at least one viscosity adjusting agent;
wherein all components of the composition are present in the composition in powder form, and add up to 100 wt.-% of the powdery composition.

2. Composition according to claim 1, wherein all components are water soluble.

3. Composition according to claim 1 or 2, wherein the composition comprises one or more further components which are selected from the list comprising fragrances, essential oils, dyes, pH adjusting agents, chelating agents, conditioning agents, active ingredients, emollients, moisturizing agents, pearlescent agents, opacifying agents, emulsifier, absorbents, preserving agents, antioxidants.

4. Composition according to at least one of the claims 1 to 3, wherein the at least one substrate is selected from lactose, silica, mannite, xylite, diatomite, cellulose and derivates thereof, monocrystalline cellulose, modified cellulose, trimethyl glycine, saccharose, monosaccharide, polysaccharide, cyclodextrin, native starch, chemically modified starch, thermal modified starch, sorbitol, polyvinylpyrrolidone, silicate, or mixtures thereof.

5. Composition according to at least one of the claims 1 to 4, wherein the at least one viscosity adjusting agent is selected from natural polymers, synthetic thickeners, ethers, alkoxylated alcohols, hydrophilic colloids or derivatives thereof, salts of monovalent or divalent cations or mixtures thereof.

6. Composition according to at least one of the claims 1 to 5, wherein the at least one surfactant is selected from isethionate, amino acid, sulfonic acid, sulfuric acid ester, alkyl sulfate, alkylether sulfate, taurate, sulfo succinate, sulfo succinamate, carboxylate, glucosides, amphoacetates, amide, alkanol amide, or mixtures thereof.

7. Method for manufacturing the composition according to at least one of claims 1 to 6, wherein the method comprises a mixing step, wherein the components of the composition are mixed together.

8. Method according to claim 7 wherein prior to the mixing step an uptake step is conducted, wherein one or more of the components being present in liquid form are up-taken onto the at least one substrate and transferred into a powder form.

9. Method for manufacturing an aqueous cleansing composition by mixing the powdery composition as claimed in at least one of the claims 1 to 6 or by mixing the powdery composition as obtained by the method as claimed in at least one of the claims 7 or 8 with water, wherein the water is selected from deionized water, purified water, mineral water or tap water, or a mixture thereof.

10. Method as claimed in claim 9 wherein the mixing of water and the powdery composition is done by shaking and/or stirring.

11. Method as claimed in at least one of claims 9 or 10, wherein the weight ratio of powdery composition: added water is 1:2.5 to 1:4.5.

12. Aqueous cleansing composition comprising the powdery composition according to at least one of the claims 1 to 6 in dissolved form or comprising the powdery composition as obtained by the method as claimed in at least one of the claims 7 or 8 in dissolved form; or aqueous cleansing composition as obtained by the method as claimed in at least one of the claims 9 to 11.

13. Aqueous cleansing composition according to claim 12, wherein the amount of the at least one surfactant is not more than 14 g wherein the amount is based on 100 g of the total aqueous cleansing composition, if the weight ratio of powdery composition : added water is 1:4.

14. Aqueous cleansing composition according to at least one of the claims 12 or 13, wherein the aqueous cleansing composition is selected from the group consisting of shampoo, 2-in-1 shampoo conditioner, shower gel, shower cream, hair and body washes, liquid hand soaps, facial cleanser or combinations thereof for human beings and for animals.

15. Aqueous cleansing composition according to at least one of the claims 12 to 14, wherein the pH of the aqueous cleansing composition is between 4.0 to 6.0 or between 5.0 to 7.5
